# EUROPEAN PATENT SPECIFICATION

(11) **EP 2 317 971 B1**
(45) Date of publication and mention of the grant of the patent: **29.07.2015**
(21) Application number: 08763777.3
(22) Date of filing: 19.03.2008
(51) Int. Cl.: A61K 8/365, A61K 8/37, A61K 8/69, A61Q 7/00

(54) **COMPOSITION FOR STIMULATING THE GROWTH OF EYELASHES, EYEBROWS AND HAIRS**
ZUSAMMENSETZUNG ZUR STIMULIERUNG DES WACHSTUMS VON WIMPERN, AUGENBRAUEN UND HAAREN
COMPOSITION POUR STIMULER LA CROISSANCE DES CILS, SOURCILS ET CHEVEUX

(43) Date of publication of application: 11.05.2011
(73) Proprietor: Caruso, Ciro, 80128 Napoli NA (IT); Sanseverino, Renato, 80122 Napoli NA (IT)
(72) Inventor: Caruso, Ciro, 80128 Napoli NA (IT); Sanseverino, Renato, 80122 Napoli NA (IT)
(74) Representative: Montelatici, Linda Anna
(86) International application number: PCT/IT2008/000183
(87) International publication number: WO 2009/116098

(56) References cited:
- WO-A-02/07731
- WO-A-03/009820
- US-A1- 2002 035 149
- US-A1- 2007 160 562
- STJERNSCHANTZ JOHAN W ET AL: "Mechanism and clinical significance of prostaglandin-induced iris pigmentation" SURVEY OF OPHTHALMOLOGY, vol. 47, no. Supplement 1, August 2002 (2002-08), pages S162-S175, XP002496471 ISSN: 0039-6257

## Description

The present invention refers to a composition for stimulating the growth, lengthening, thickening and darkening of eyelashes, eyebrows and hairs.

It is known that some dermatologic diseases result in a loss of eyelashes up to the point of total loss thereof. For example, hypotrichosis illnesses or the loss of eyelashes due to local destructive processes are known, such as ulcerative belpharitis, trachoma or caustic injuries.

Some cosmetic products for the treatment of eyelashes based on plant extracts are known, for example castor oil or soya lecithin.

These products are effective in improving the robustness and the aesthetic aspect of the eyelashes, thus making them shinier and thicker, however, their usefulness to support the growth in cases of hypotrichosis of the eyelashes and/or of the eyebrows has shown to be rather limited.

It is further known that some eye drops for the treatment of glaucoma contain prostaglandin of the PGF2 group, having the basic structure of fluprostenol.

For example, US2002/0035149 discloses 15-keto latanoprost and other 15-keto prostaglandin analogues as intraocular pressure reducing agents.

WO02/07731 discloses a composition for treatment of ocular hypertension and glaucoma, comprising 15-keto prostaglandin compounds.

This prostaglandin group comprises, among others, molecules known under the commercial names Bimatoprost, Travoprost, Latanoprost and Unoprostone. Beside of a hypotensive effect on the intraocular pressure, these prostaglandins have several side effects, among which the growth, blackening and thickening of the eyelashes of the treated eye are mentioned. Particularly the Travoprost molecule, or fluprostenol isopropyl ester, has shown to be particularly effective in re-growing, thickening and lengthen eyelashes in patients affected by hypotrichosis , madarosis, alopecia areata.

WO03/009820 discloses methods and compositions for the promotion of hair growth in mammals, comprising PGF_{2α} analogues.

US2007/160562 discloses improved cosmeceuticals capable of stimulating the growth of hair, i.e. natural eyelashes, in a human subject.

However, the seriousness of other unwanted effects produced by these molecules makes their use impracticable or strongly inconvenient in the treatment of the loss of eyelashes in the above-mentioned diseases. These further unwanted effects include in fact conjunctival hyperemia, eye burning, darkening of the iris and dark colouring of the edge of the eyelid. Particularly these last two effects are intolerable for the user because these result to be permanent. Especially the colouring of the edge of the eyelid, even mentioned in the package leaflet of the medicines comprising Bimatoprost, Travoprost, Latanoprost, is considered a practicably unavoidable effect when fluprostenol molecules are used, and turns out to be particularly undesired because clearly visible.

Recently also some cosmetic products for the treatment of eyelashes and hair have been proposed, containing one of the above-mentioned prostaglandins. For example, in the United States a product for cosmetic use has been launched that favours the growth of eyelashes, applicable as eye-liner and containing the active ingredient Bimatoprost. However it is suspected that the prolonged use of this product could cause, beside of the above-mentioned side effects, also those of edema of the retina and the reduction of sight.

It is therefore object of the present invention to provide a composition for stimulating the growth and the thickening of eyelashes, which is free from the disadvantages of the known compositions.Said object is achieved with a composition for use for stimulating the growth of eyelashes and/or eyebrows and/or hair in a patient in need thereof comprising a compound of formula (I): 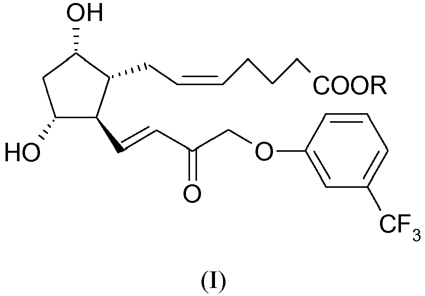 wherein R is isopropyl

It has surprisingly been found in fact that the above-identified compound of formula (I) do not produce the above-mentioned unwanted effects typical of the other molecules having the basic skeleton of fluprostenol, but maintain the desired effect to favour the growth and thickening of eyelashes and eyebrows.

In particular, the compounds of formula (I) have the same efficacy as Travoprost in letting re-grow, lengthen, darken or thicken the eyelashes, but without darken the edge of the eyelid or colouring the iris, not even temporarily.

The compositions containing the compound of formula (I) according to the invention may be in the form of mascara, eyeliner or ointments, to be applied with a brush or a cotton tipped applicator onto the areas where the eyelashes are missing.

Further, it has been found that the compositions containing a compound of formula (I) as active ingredient do not cause burning even if occasionally getting into contact with the eye.

In a second aspect thereof the invention refers to the use of a compound of formula (I) for the preparation of a composition intended to stimulate the growth of eyelashes, eyebrows and/or hair.

Further advantages and characteristics of the composition according to the present invention will become clear to those skilled in the art from the following detailed and non-limiting description of some embodiments thereof.

The compositions according to the invention comprise a compound of formula (I): 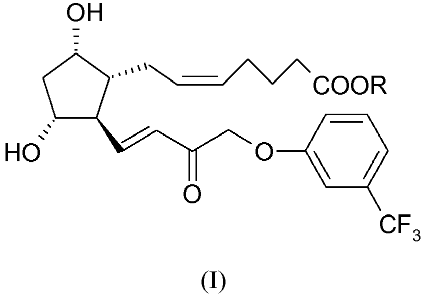 wherein R is isopropyl.

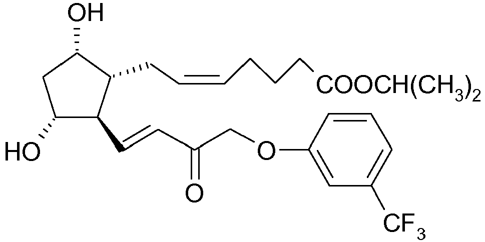

This compound is defined 15-keto fluprostenol isopropyl ester or isopropyl (±)15-oxo-9a,11a-dihydroxy-16,16-(3-(trifluoromethyl)phenoxy)-17,18,19,20-tetranor-prosta-5Z,13E-dien-1-oate.

It has in fact been found that the compound given above is particularly suitable for the making of compositions for use in re-growing, lengthening, thickening and darkening the eyelashes and are harmless as far as unwanted side effects regarding the darkening of the skin of the eyelids and of the iris are concerned, as results from the example reported below.

The reasons for the surprising efficacy of the compound of formula (I) in letting the eyelashes re-grow, lengthen and thicken without colouring the iris or the edge of the eyelid are not yet completely defined.

Without any intention to restrict the invention to a theory, the inventors however believe that a possible explanation of the efficacy of the compounds of formula (I) may be a reduced affinity and selectivity of the compounds of formula (I) for the FP receptor (prostagladin receptor), determined by the presence of the C-15 keto functional group.

It has in fact been shown that there is an increased melanogenosis with a consequent darkening of the skin introduced by the prostagladins having a C15-hydroxy group, among which e.g. the active ingredient Travoporst can be mentioned.

It is therefore believed that the compounds having a C-15 keto functional group, although maintaining a weak hypostensive intra-ocular response, do not have any capacity to introduce melangenesis, which is the cause of the darkening of the skin around the eyes.

The compositions according to the present invention can be advantageously produced in the form of a mascara or an eyeliner, and hence contain one or more further additives chosen among solvents, preservatives, viscoelastic substances, antimycotics, antimicrobials, pigments, adhesive substances and cosmetic agents having beneficiary properties for the eyelashes and eyebrows.

For example, as preservatives suitable for the composition according to the present invention benzoic acid, propionic acid, salicylic acid, sorbic acid, formaldehyde, o-phenylphenol, zinc salt, sulfites, bisulfite, sodium iodate, clorobutanol, p-hydroxybenzoic acid, dehydroacetic acid, formic acid, undecanoic acid, benzyl alcohol, clorofene may be mentioned. Among the viscoelastic substances hyaluronic acid, polyethylenglycol, polyvinylalcohol, methylcellulose, carboxymethylcellulose, hydroxymethylcellulose may be mentioned. As adhesive substance glycerol may be used, while as antimycotic, antimicrobial and antifungal agents benzalconium chloride may be used.

According to the present embodiment of the invention, the compound of formula (I) is preferably present in the composition for the treatment of eyelashes or eyebrows in quantities in the range from 5x10⁻¹g/ml to 5x10⁻⁷g/ml. In a particularly advantageous way, the concentration of the compound according to formula (I) in the composition for the treatment of eyelashes and eyebrows is 50 µg/ml.

The compounds of formula (I) may also be used in the form of a hair lotion, useful to let re-grow or lengthen or thicken or darken the hair without blackening the skin with which it gets into contact.

Thus the present inventions refers also to a composition comprising one or more compounds of formula (I), in the form of a hair lotion. This composition for the treatment of hair may hence also comprise one or more further additives, as for example: plant lipoproteins (PEG), thickeners, perfumes, buffers, antistatic agents, humectants and vitamins.

The efficacy of the composition for the treatment of eyelashes and eyebrows according to the invention is described by means of the following example, that has a merely illustrative and non-limiting function.

### EXAMPLE 1

Three hypotonic solutions (A, B, C) buffered at pH 7.2 were prepared, containing:

| | |
|---|---|
| Active ingredient | 50 µg/ml |
| Hyaluronic acid | 0.003 g/ml |
| N-hydroxymethyl glycine | 0.00002 g/ml |
| Sodium edatate | 0.001 g/ml |

wherein: in solution A the active ingredient was the compound of formula (I) with R= isopropyl;
in solution B the active ingredient was the compound of formula (I) with R= hydrogen;
in solution C the active ingredient was the commercial active ingredient Travoprost.

A number of 34 patients affected by hypotrichosis, ulcerative belpharitis or madarosis were examined, of which 18 female and 16 male having an age between 18 and 67 years, that were divided into three groups.

The first group, consisting of 6 female and 5 male patients, was asked to use the above-defined solution A.

The second group, also consisting of 6 female and 5 male patients, was asked to use the above-defined solution B.

The third group, consisting of 6 female and 6 male patients, was asked to use the above-defined solution C.

The solutions were applied onto the edges of the eyelid in those patients without any eyelashes, by means of a cotton tipped applicator, and onto the eyelashes of those patients with a loss of about 50% of the eyelashes themselves, by means of a soft mascara-like brush.

The solutions were applied two times a day, in the morning after wakening and in the evening before going to bed.

The controls to verify the growth of the eyelashes was performed after 2 and 4 month from the begin of the treatment, and subsequently 8 month after the end of the treatment. The eyelashes were measured with a preset gauge and the results are reported in the following.

Already after one month of treatment 87% of the patients of the three groups showed a restarting of the growth of the eyelashes, in those who had suffered a loss, or a thickening in those who instead already had some eyelashes; all the patients had the benefit of a lengthening of the eyelashes and a darkening in those subjects with eyelashes of a light colour.

Four month after the beginning of the treatment 100% of the patients showed the above-reported improvements.

The control performed 8 month after the end of the treatment showed a regression of the effects in 35% of the cases, distributed as follows: 9% of the cases of the first group; 13% of the cases of the second group; and 13% of the cases of the third group.

The patients of the first and the second group have not complained about subjective side effects and did not show objective visible signs.

In the third group instead both, subjective symptoms and visible signs, were evident.

In particular, 25% of the patients of the third group showed objective signs controlled by means of a slit lamp distributed as follows: about 80% of the 25% of the patients showed conjunctival hyperemia, increase in lacrimation, conjunctival reddening, edema of the eyelid; 33% of the 25% showed a darkening of the skin around the eyes.

From the subjective point of view the whole third group complained about eye irritation, photophobia, ocular itching and burning.

## Claims

1. A composition for use for stimulating the growth of eyelashes and/or eyebrows and/or hair in a patient in need thereof, **characterized by** comprising a compound of formula (I): 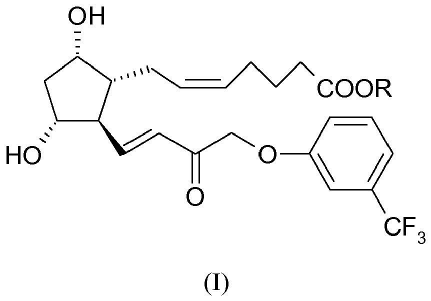 wherein R is isopropyl.

2. A composition for use according to claim 1, **characterized by** being in the form of a mascara or an eyeliner.

3. A composition for use according to claim 2, **characterized in that** said compound of formula (I) has a concentration in the range from 5x10⁻¹g/ml to 5x10⁻⁷ g/ml.

4. A composition for use according to claim 3, **characterized in that** said compound of formula (I) has a concentration of 50 µg/ml.

5. A composition for use according to claim 1, **characterized by** being in the form of a hair lotion.

6. A composition for use according to any one of claims 1 to , **characterized by** comprising one or more further additives chosen among solvents, viscoelastic substances, antimycotics, antimicrobials, pigments, adhesive substances and cosmetic agents having beneficiary properties for eyelashes and eyebrows.

## Patentansprüche

1. Eine Zusammensetzung zur Verwendung für die Stimulierung des Wachstums von Wimpern und/oder Augenbrauen und/oder Haaren eines desselben bedürftigen Patienten, **dadurch gekennzeichnet, dass** sie eine Verbindung der Formel (I) umfasst: 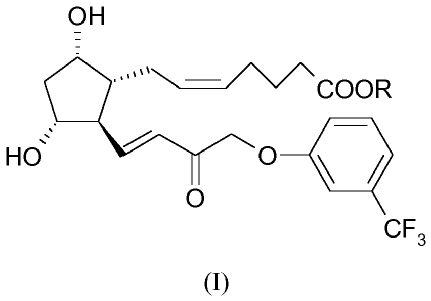 wobei R Isopropyl ist.

2. Eine Zusammensetzung zur Verwendung gemäß Anspruch 1, **gekennzeichnet durch** die Form einer Wimperntusche oder eines Augenkonturenstifts.

3. Eine Zusammensetzung zur Verwendung gemäß Anspruch 2, **dadurch gekennzeichnet, dass** die besagte Verbindung der Formel (I) eine Konzentration in dem Bereich von 5x10⁻¹ g/ml bis 5x10⁻⁷ g/ml hat.

4. Eine Zusammensetzung zur Verwendung gemäß Anspruch 3, **dadurch gekennzeichnet, dass** die besagte Verbindung der Formel (I) eine Konzentration von 50 µg/ml hat.

5. Eine Zusammensetzung zur Verwendung gemäß Anspruch 1, **gekennzeichnet durch** die Form einer Haarlotion.

6. Eine Zusammensetzung zur Verwendung gemäß einem der Ansprüche 1 bis 4, **dadurch gekennzeichnet, dass** sie ein oder mehrere weitere Additive umfasst, ausgewählt aus Lösungsmitteln, viskoelastischen Substanzen, Antimykotika, Antimikrobiotika, Pigmenten, Haftmitteln und Kosmetika mit für Wimpern und Augenbrauen günstigen Eigenschaften.

## Revendications

1. Composition pour une utilisation dans la stimulation de la croissance de cils et/ou de sourcils et/ou de cheveux chez un patient en ayant besoin, **caractérisée en ce qu'**elle comprend un composé de formule (I) : 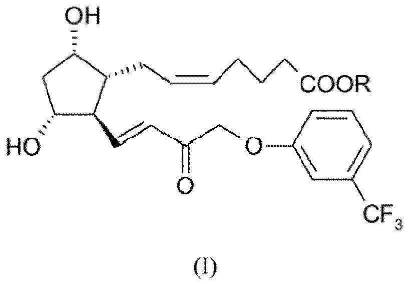 dans laquelle R est un groupe isopropyle.

2. Composition pour une utilisation selon la revendication 1, **caractérisée en ce qu'**elle est sous la forme d'un mascara ou d'un eyeliner.

3. Composition pour une utilisation selon la revendication 2, **caractérisée en ce que** ledit composé de formule (I) a une concentration comprise dans la plage allant de 5x10⁻¹ à 5x10⁻⁷ g/ml.

4. Composition pour une utilisation selon la revendication 3, **caractérisée en ce que** ledit composé de formule (I) a une concentration de 50 µg/ml.

5. Composition pour une utilisation selon la revendication 1, **caractérisée en ce qu'**elle est sous la forme d'une lotion capillaire.

6. Composition pour une utilisation selon l'une quelconque des revendications 1 à 4, **caractérisée en ce qu'**il comprend un ou plusieurs autres additifs choisis parmi les solvants, les substances viscoélastiques, les antimycotiques, les antimicrobiens, les pigments, les substances adhésives et les agents cosmétiques ayant des propriétés avantageuses pour les cils et les sourcils.
